# EUROPEAN PATENT SPECIFICATION

(11) **EP 4 395 679 B1**
(45) Date of publication and mention of the grant of the patent: **21.10.2026**
(21) Application number: 22786093.9
(22) Date of filing: 25.08.2022
(51) Int. Cl.: A61B 34/30, A61B 34/37, A61B 18/14, A61B 34/00

(54) **ARTICULATION DRIVE ASSEMBLIES FOR SURGICAL INSTRUMENTS SUCH AS FOR USE IN SURGICAL ROBOTIC SYSTEMS**
GELENKANTRIEBSANORDNUNGEN FÜR CHIRURGISCHE INSTRUMENTE WIE ETWA ZUR VERWENDUNG IN CHIRURGISCHEN ROBOTERSYSTEMEN
ENSEMBLES D'ENTRAÎNEMENT D'ARTICULATION POUR INSTRUMENTS CHIRURGICAUX TELS QUE CEUX DESTINÉS À ÊTRE UTILISÉS DANS DES SYSTÈMES CHIRURGICAUX ROBOTISÉS

(30) Priority: 30.08.2021 US 202163238469 P
(43) Date of publication of application: 10.07.2024
(73) Proprietor: Covidien LP, Mansfield, MA 02048 (US)
(72) Inventor: HEILIGER, Zachary S., Minneapolis, Minnesota 55432 (US)
(74) Representative: Zimmermann & Partner Patentanwälte mbB
(86) International application number: PCT/IB2022/057986
(87) International publication number: WO 2023/031742

(56) References cited:
- US-A1- 2020 237 455
- US-A1- 2020 261 167
- US-A1- 2020 337 787

## Description

### BACKGROUND

### 1. Technical Field

This disclosure relates generally to surgical robotic systems and, more particularly, to articulation drive assemblies for surgical instruments and surgical instruments incorporating the same for use in surgical robotic systems.

### 2. Background of Related Art

Surgical robotic systems are increasingly utilized in various different surgical procedures. Some surgical robotic systems include a console supporting a robotic arm. One or more different surgical instruments may be configured for use with the surgical robotic system and are selectively mountable to the robotic arm. The robotic arm provides one or more inputs to the mounted surgical instrument(s) to enable operation of the mounted surgical instrument(s). Such mounted surgical instrument(s) may include, for example, scalpels, graspers, clip appliers, staplers, energy-based devices (e.g., for tissue ablation, tissue sealing, tissue dissection, etc.), and/or visualization devices (e.g., endoscopes), etc. US 2020/0337787 A1 discloses a surgical instrument articulation assembly including a lead screw sub-assembly including four lead screws. A first gear sub-assembly includes first and second gears coupled to the first and third lead screws. A second gear sub-assembly includes third and fourth gears coupled to the second and fourth lead screws, respectively, a first compound gear including a distal gear coupling the first and second gears with one another, and a proximal gear coupled with a first coupling gear disposed about a first input. The third gear sub-assembly includes a second compound gear including a distal gear coupling the third and fourth gears with one another, and a proximal gear coupled with a second coupling gear disposed about a second input. A rotational input provided to the first or second input respectively rotates the first and third lead screws and or the second and fourth lead screws.

### SUMMARY

As used herein, the term "distal" refers to the portion that is being described which is farther from an operator (whether a human surgeon or a surgical robot arm), while the term "proximal" refers to the portion that is being described which is closer to the operator. Terms including "generally," "about," "substantially," and the like, as utilized herein, are meant to encompass variations, e.g., manufacturing tolerances, material tolerances, use and environmental tolerances, measurement variations, design variations, and/or other variations, up to and including plus or minus 10 percent. Further, to the extent consistent, any or all of the aspects detailed herein may be used in conjunction with any or all of the other aspects detailed herein.

The invention is defined by claims 1 and 11. Further embodiments of the invention are defined by the dependent claims.

Provided in accordance with aspects of this disclosure is an articulation assembly for a surgical instrument. The articulation assembly includes a proximal housing, first and second coupling gears, first and second input shafts, and first, second, third, and fourth output gears. The proximal housing includes a central cylindrical body disposed therein and first, second, third, and fourth internal cavities defined therein and arranged about the central cylindrical body. The first and second coupling gears are coaxially disposed about the central cylindrical body within the proximal housing with the first coupling gear more-distally disposed and the second coupling gear more-proximally disposed. The first and second input shafts are disposed at least partially within the first and second cavities, respectively, and include proximal receivers extending proximally from the proximal housing that are configured to receive first and second rotational inputs, respectively. The first and second input shafts also include respective first and second gears, wherein the first gear is disposed more-distally and in meshed engagement with the first coupling gear, and wherein the second gear is disposed more-proximally and in meshed engagement with the second coupling gear. Thus, the first rotational input rotates the first input shaft to thereby rotate the first coupling gear and the second rotational input rotates the second input shaft to thereby rotate the second coupling gear. The first, second, third, and fourth output gears are disposed within the proximal housing wherein the first output gear is disposed within the first cavity distally of the first input shaft and in meshed engagement with the first coupling gear, the second output gear is disposed within the second cavity distally of the second input shaft and in meshed engagement with the second coupling gear, the third output gear is disposed within the third cavity and in meshed engagement with the first coupling gear, and the fourth output gear is disposed within the fourth cavity and in meshed engagement with the second coupling gear. The first rotational input rotates the first and third output gears in the same direction with equal magnitude and the second rotational input rotates the second and fourth output gears in the same direction with equal magnitude.

In an aspect of this disclosure, the first and third output gears are diagonally opposite one another and wherein the second and fourth output gears are diagonally opposite one another.

In another aspect of this disclosure, the first and second coupling gears are compound gears each including a major gear and a minor gear. In such aspects, one of the major gears or the minor gears of the first and second coupling gears are disposed in meshed engagement with the first and second gears of the first and second input shafts, respectively. The other of the major gears or the minor gears of the first and second coupling gears are disposed in meshed engagement with the first and third output gears and the second and fourth output gears, respectively.

In still another aspect of this disclosure, first, second, third, and fourth lead screws extend proximally into the first, second, third, and fourth cavities and are rotatably fixed relative to the first, second, third, and fourth output gears within the proximal housing such that rotation of one of the first, second, third, or fourth output gears rotates a corresponding one of the first, second, third, or fourth lead screws.

In yet another aspect of this disclosure, first, second, third, and fourth collars are threadingly engaged about the first, second, third, and fourth lead screws, respectively, such that rotation of one of the first, second, third, or fourth lead screws translates a corresponding one of the first, second, third, or fourth collars therealong.

In still yet another aspect of this disclosure, first, second, third, and fourth articulation cables are connected to the first, second, third, and fourth collars, respectively, such that translation of one of the first, second, third, or fourth collars tensions or de-tensions a corresponding one of the first, second, third, or fourth articulation cables.

In another aspect of this disclosure, a proximal shaft portion of each of the first, second, third, and fourth lead screws extends proximally into a corresponding one of the first, second, third, or fourth cavities, and supports a corresponding one of the first, second, third, or fourth output gears thereon within the proximal housing.

In yet another aspect of this disclosure, the proximal shaft portion of each of the first, second, third, and fourth lead screws defines a non-cylindrical configuration and each of the first, second, third, and fourth output gears defines an aperture shaped at least partially complementary to the proximal shaft portion of a corresponding one of the first, second, third, and fourth lead screws to thereby rotatably fix the first, second, third, and fourth output gears about the first, second, third, and fourth lead screws, respectively.

A surgical instrument provided in accordance with this disclosure includes a housing, a shaft extending distally from the housing and including an articulating section, an end effector assembly extending distally from the shaft, and first, second, third, and fourth articulation cables operably coupled to the articulating section and extending proximally through the shaft into the housing to proximal end portions thereof. The surgical instrument further includes the articulation assembly according to any of the aspects detailed herein, wherein the first, second, third, and fourth articulation cables are operably coupled to the first, second, third, and fourth output gears, respectively.

Another articulation assembly for a surgical instrument provided in accordance with aspects of this disclosure includes a proximal housing, first and second input shafts, first, second, third, and fourth lead screws, and first and second compound coupling gears. The proximal housing includes a central cylindrical body disposed therein and first, second, third, and fourth internal cavities defined therein and arranged about the central cylindrical body. The first and second input shafts are configured to receive first and second rotational inputs and include respective first and second gears wherein the first gear is disposed more-distally within the proximal housing and the second coupling gear is disposed more-proximally within the proximal housing. The first, second, third, and fourth lead screws extend proximally into the first, second, third, and fourth cavities, respectively and include first, second, third, and fourth output gears, respectively, fixedly disposed thereabout within the respective first, second, third, and fourth cavities. The first output gear is disposed distally of the first gear of the first input shaft and the second output gear is disposed distally of the second gear of the second input shaft. The first and second compound coupling gears are coaxially disposed about the central cylindrical body within the proximal housing with the first coupling gear disposed more-distally and the second coupling gear disposed more-proximally. The first compound coupling gear is disposed in meshed engagement with the first gear, the first output gear, and the third output gear such that rotation of the first input shaft rotates the first and third lead screws in the same direction with equal magnitude. The second compound coupling gear is disposed in meshed engagement with the second gear, the second output gear, and the fourth output gear such that rotation of the second input shaft rotates the second and fourth lead screws in the same direction with equal magnitude.

In an aspect of this disclosure, the first and third output gears are diagonally opposite one another and the second and fourth output gears are diagonally opposite one another.

In another aspect of this disclosure, the first and second compound coupling gears each include a major gear and a minor gear. In such aspects, one of the major gears or the minor gears of the first and second compound coupling gears is disposed in meshed engagement with the first and second gears of the first and second input shafts, respectively, and/or the other of the major gears or the minor gears of the first and second compound coupling gears are disposed in meshed engagement with the first and third output gears and the second and fourth output gears, respectively.

In yet another aspect of this disclosure, first, second, third, and fourth collars are threadingly engaged about the first, second, third, and fourth lead screws, respectively, such that rotation of one of the first, second, third, or fourth lead screws translates a corresponding one of the first, second, third, or fourth collars therealong.

In still another aspect of this disclosure, first, second, third, and fourth articulation cables are connected to the first, second, third, and fourth collars, respectively, such that translation of one of the first, second, third, or fourth collars tensions or de-tensions a corresponding one of the first, second, third, or fourth articulation cables.

In still yet another aspect of the present disclosure, a proximal shaft portion of each of the first, second, third, and fourth lead screws defines a non-cylindrical configuration and each of the first, second, third, and fourth output gears defines an aperture shaped at least partially complementary to the proximal shaft portion of a corresponding one of the first, second, third, and fourth lead screws to thereby rotatably fix the first, second, third, and fourth output gears about the first, second, third, and fourth lead screws, respectively.

### BRIEF DESCRIPTION OF THE DRAWINGS

The above and other aspects and features of this disclosure will become more apparent in view of the following detailed description when taken in conjunction with the accompanying drawings wherein like reference numerals identify similar or identical elements.
FIG. 1 is a schematic illustration of a surgical robotic system including a control tower, a console, and one or more surgical robotic arms according to aspects of this disclosure;
FIG. 2 is a perspective view of a surgical robotic arm of the surgical robotic system of FIG. 1 according to aspects of this disclosure;
FIG. 3 is a perspective view of a setup arm with the surgical robotic arm of the surgical robotic system of FIG. 1 according to aspects of this disclosure;
FIG. 4 is a schematic diagram of a computer architecture of the surgical robotic system of FIG. 1 according to aspects of this disclosure;
FIG. 5 is a perspective view of a surgical instrument provided in accordance with the present disclosure configured for mounting on a robotic arm of a surgical robotic system such as the surgical robotic system of FIG. 1;
FIGS. 6A and 6B are front and rear perspective views, respectively, of a proximal portion of the surgical instrument of FIG. 5, with an outer shell removed;
FIG. 7 is a front perspective view of the proximal portion of the surgical instrument of FIG. 5 with the outer shell and additional internal components removed;
FIG. 8 is a side view of the articulation sub-assembly of the surgical instrument of FIG. 5;
FIGS. 9A and 9B are front and rear perspective views, respectively, of the articulation sub-assembly of FIG. 8 with the distal support plate removed;
FIGS. 10A-10C are longitudinal, cross-sectional views through a center, right portion, and left portion, respectively, of the articulation sub-assembly of FIG. 8 with the distal support plate removed;
FIGS 11A and 11B are perspective views of the housing cap and housing base, respectively, of the proximal housing of the articulation sub-assembly of FIG. 8;
FIGS. 12A and 12B are perspective views of the respective first and second input shafts of the articulation sub-assembly of FIG. 8;
FIG. 13 is a perspective view of one of the two identical coupling gears of the articulation sub-assembly of FIG. 8;
FIG. 14 is a perspective view of one of the four identical output gears of the articulation sub-assembly of FIG. 8;
FIGS. 15A and 15B are side views of right-hand pitched and left-hand pitched lead screws, respectively, of the articulation sub-assembly of FIG. 8; and
FIGS. 16A and 16B are perspective views of collars for the respective right-hand pitched and left-hand pitched lead screws of the articulation sub-assembly of FIG. 8.

### DETAILED DESCRIPTION

This disclosure provides articulation drive assemblies for surgical instruments and surgical instruments incorporating the same. As described in detail below, the articulation drive assemblies and surgical instruments of this disclosure are configured for use with a surgical robotic system, which may include, for example, a surgical console, a control tower, and one or more movable carts having a surgical robotic arm coupled to a setup arm. The surgical console receives user input through one or more interface devices, which are interpreted by the control tower as movement commands for moving the surgical robotic arm. The surgical robotic arm includes a controller, which is configured to process the movement command and to generate a torque command for activating one or more actuators of the robotic arm, which, in turn, move the robotic arm in response to the movement command. Those skilled in the art will understand that this disclosure, although described in connection with surgical robotic systems, may also be adapted for use with endoscopic surgical instruments and/or open surgical instruments.

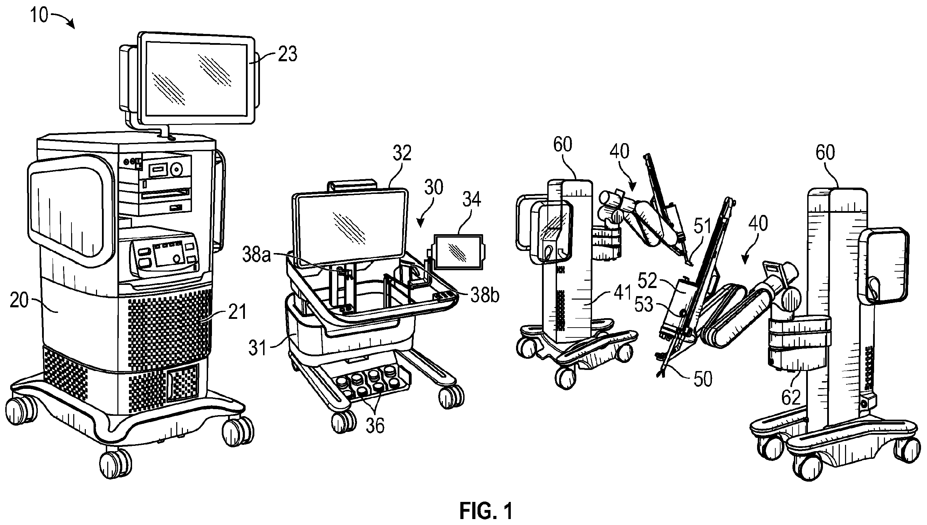

With reference to FIG. 1, a surgical robotic system 10 includes a control tower 20, which is connected to components of the surgical robotic system 10 including a surgical console 30 and one or more robotic arms 40. Each of the robotic arms 40 includes a surgical instrument 50 removably coupled thereto. Each of the robotic arms 40 is also coupled to a movable cart 60.

The one or more surgical instruments 50 may be configured for use during minimally invasive surgical procedures and/or open surgical procedures. In aspects, one of the surgical instruments 50 may be an endoscope, such as an endoscope camera 51, configured to provide a video feed for the clinician. In further aspects, one of the surgical instruments 50 may be an energy-based surgical instrument such as, for example, an electrosurgical forceps or ultrasonic sealing and dissection instrument configured to seal tissue by grasping tissue between opposing structures and applying electrosurgical energy or ultrasonic energy, respectively, thereto. In yet further aspects, one of the surgical instruments 50 may be a surgical stapler including a pair of jaws configured to clamp tissue, deploy a plurality of tissue fasteners, e.g., staples, through the clamped tissue, and/or to cut the stapled tissue.

One of the robotic arms 40 may include a camera 51 configured to capture video of the surgical site. The surgical console 30 includes a first display 32, which displays a video feed of the surgical site provided by camera 51 of the surgical instrument 50 disposed on the robotic arms 40, and a second display 34, which displays a user interface for controlling the surgical robotic system 10. The first and second displays 32 and 34 are touchscreens allowing for displaying various graphical user inputs.

The surgical console 30 also includes a plurality of user interface devices, such as foot pedals 36 and a pair of handle controllers 38a and 38b which are used by a user to remotely control robotic arms 40. The surgical console further includes an armrest 33 used to support clinician's arms while operating the handle controllers 38a and 38b.

The control tower 20 includes a display 23, which may be a touchscreen, and outputs on the graphical user interfaces (GUIs). The control tower 20 also acts as an interface between the surgical console 30 and one or more robotic arms 40. In particular, the control tower 20 is configured to control the robotic arms 40, such as to move the robotic arms 40 and the corresponding surgical instrument 50, based on a set of programmable instructions and/or input commands from the surgical console 30, in such a way that robotic arms 40 and the surgical instrument 50 execute a desired movement sequence in response to input from the foot pedals 36 and the handle controllers 38a and 38b.

Each of the control tower 20, the surgical console 30, and the robotic arm 40 includes a respective computer 21, 31, 41. The computers 21, 31, 41 are interconnected to each other using any suitable communication network based on wired or wireless communication protocols. The term "network," whether plural or singular, as used herein, denotes a data network, including, but not limited to, the Internet, Intranet, a wide area network, or a local area networks, and without limitation as to the full scope of the definition of communication networks as encompassed by the present disclosure. Suitable protocols include, but are not limited to, transmission control protocol/internet protocol (TCP/IP), datagram protocol/internet protocol (UDP/IP), and/or datagram congestion control protocol (DCCP). Wireless communication may be achieved via one or more wireless configurations, e.g., radio frequency, optical, Wi-Fi, Bluetooth (an open wireless protocol for exchanging data over short distances, using short length radio waves, from fixed and mobile devices, creating personal area networks (PANs), ZigBee^{®} (a specification for a suite of high level communication protocols using small, low-power digital radios based on the IEEE 122.15.4-2003 standard for wireless personal area networks (WPANs)).

The computers 21, 31, 41 may include any suitable processor (not shown) operably connected to a memory (not shown), which may include one or more of volatile, non-volatile, magnetic, optical, or electrical media, such as read-only memory (ROM), random access memory (RAM), electrically-erasable programmable ROM (EEPROM), non-volatile RAM (NVRAM), or flash memory. The processor may be any suitable processor (e.g., control circuit) adapted to perform the operations, calculations, and/or set of instructions described in the present disclosure including, but not limited to, a hardware processor, a field programmable gate array (FPGA), a digital signal processor (DSP), a central processing unit (CPU), a microprocessor, and combinations thereof. Those skilled in the art will appreciate that the processor may be substituted for by using any logic processor (e.g., control circuit) adapted to execute algorithms, calculations, and/or set of instructions described herein.

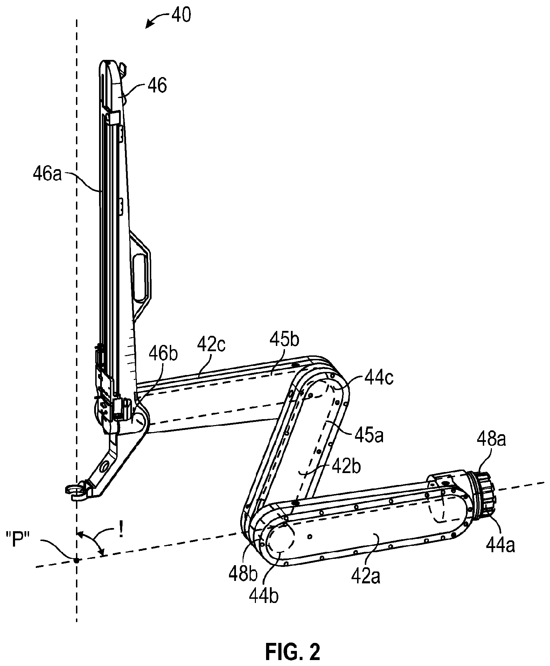

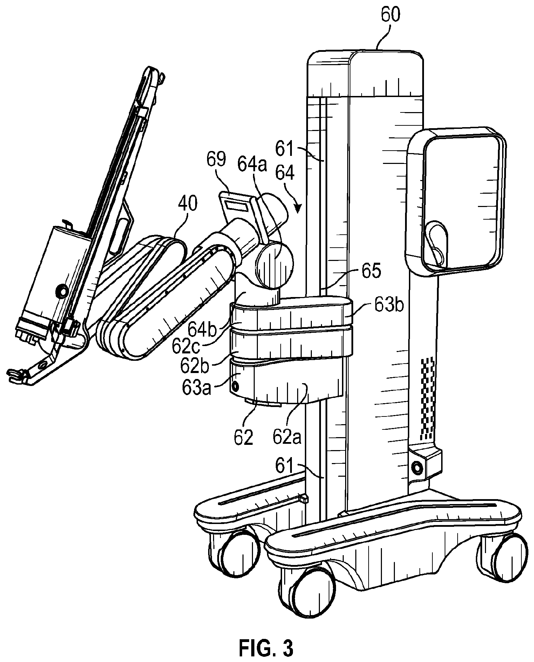

With reference to FIG. 2, each of the robotic arms 40 may include a plurality of links 42a, 42b, 42c, which are interconnected at joints 44a, 44b, 44c, respectively. The joint 44a is configured to secure the robotic arm 40 to the movable cart 60 and defines a first longitudinal axis. With reference to FIG. 3, the movable cart 60 includes a lift 61 and a setup arm 62, which provides a base for mounting of the robotic arm 40. The lift 61 allows for vertical movement of the setup arm 62. The movable cart 60 also includes a display 69 for displaying information pertaining to the robotic arm 40.

The setup arm 62 includes a first link 62a, a second link 62b, and a third link 62c, which provide for lateral maneuverability of the robotic arm 40. The links 62a, 62b, 62c are interconnected at joints 63a and 63b, each of which may include an actuator (not shown) for rotating the links 62b and 62b relative to each other and the link 62c. In particular, the links 62a, 62b, 62c are movable in their corresponding lateral planes that are parallel to each other, thereby allowing for extension of the robotic arm 40 relative to the patient (e.g., surgical table). In embodiments, the robotic arm 40 may be coupled to the surgical table (not shown). The setup arm 62 includes controls 65 for adjusting movement of the links 62a, 62b, 62c as well as the lift 61.

The third link 62c includes a rotatable base 64 having two degrees of freedom. In particular, the rotatable base 64 includes a first actuator 64a and a second actuator 64b. The first actuator 64a is rotatable about a first stationary arm axis which is perpendicular to a plane defined by the third link 62c and the second actuator 64b is rotatable about a second stationary arm axis which is transverse to the first stationary arm axis. The first and second actuators 64a and 64b allow for full three-dimensional orientation of the robotic arm 40.

With reference again to FIG. 2, the robotic arm 40 also includes a holder 46 defining a second longitudinal axis and configured to receive an IDU 52 (FIG. 1). The IDU 52 is configured to couple to an actuation mechanism of the surgical instrument 50 and the camera 51 and is configured to move (e.g., rotate) and actuate the instrument 50 and/or the camera 51. IDU 52 transfers actuation forces from its actuators to the surgical instrument 50 to actuate components (e.g., end effectors) of the surgical instrument 50. The holder 46 includes a sliding mechanism 46a, which is configured to move the IDU 52 along the second longitudinal axis defined by the holder 46. The holder 46 also includes a joint 46b, which rotates the holder 46 relative to the link 42c.

The robotic arm 40 also includes a plurality of manual override buttons 53 disposed on the IDU 52 and the setup arm 62, which may be used in a manual mode. The clinician may press one or the buttons 53 to move the component associated with the button 53.

The joints 44a and 44b include an actuator 48a and 48b configured to drive the joints 44a, 44b, 44c relative to each other through a series of belts 45a and 45b or other mechanical linkages such as a drive rod, a cable, or a lever and the like. In particular, the actuator 48a is configured to rotate the robotic arm 40 about a longitudinal axis defined by the link 42a.

The actuator 48b of the joint 44b is coupled to the joint 44c via the belt 45a, and the joint 44c is in turn coupled to the joint 46c via the belt 45b. Joint 44c may include a transfer case coupling the belts 45a and 45b, such that the actuator 48b is configured to rotate each of the links 42b, 42c and the holder 46 relative to each other. More specifically, links 42b, 42c, and the holder 46 are passively coupled to the actuator 48b which enforces rotation about a remote center point "P" which lies at an intersection of the first axis defined by the link 42a and the second axis defined by the holder 46. Thus, the actuator 48b controls the angle θ between the first and second axes allowing for orientation of the surgical instrument 50. Due to the interlinking of the links 42a, 42b, 42c, and the holder 46 via the belts 45a and 45b, the angles between the links 42a, 42b, 42c, and the holder 46 are also adjusted in order to achieve the desired angle θ. In embodiments, some or all of the joints 44a, 44b, 44c may include an actuator to obviate the need for mechanical linkages.

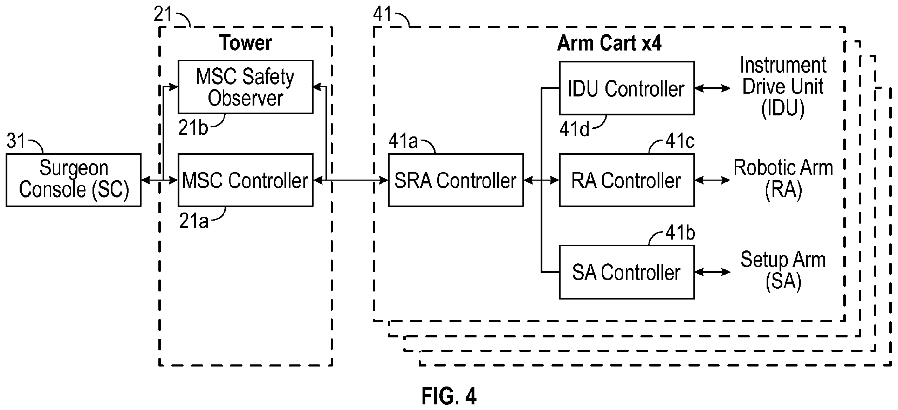

With reference to FIG. 4, each of the computers 21, 31, 41 of the surgical robotic system 10 may include a plurality of controllers, which may be embodied in hardware and/or software. The computer 21 of the control tower 20 includes a controller 21a and safety observer 21b. The controller 21a receives data from the computer 31 of the surgical console 30 about the current position and/or orientation of the handle controllers 38a and 38b and the state of the foot pedals 36 and other buttons. The controller 21a processes these input positions to determine desired drive commands for each joint of the robotic arm 40 and/or the IDU 52 and communicates these to the computer 41 of the robotic arm 40. The controller 21a also receives back the actual joint angles and uses this information to determine force feedback commands that are transmitted back to the computer 31 of the surgical console 30 to provide haptic feedback through the handle controllers 38a and 38b. The handle controllers 38a and 38b include one or more haptic feedback vibratory devices that output a haptic feedback. The safety observer 21b performs validity checks on the data going into and out of the controller 21a and notifies a system fault handler if errors in the data transmission are detected to place the computer 21 and/or the surgical robotic system 10 into a safe state.

The computer 41 includes a plurality of controllers, namely, a main cart controller 41a, a setup arm controller 41b, a robotic arm controller 41c, and an instrument drive unit (IDU) controller 41d. The main cart controller 41a receives and processes joint commands from the controller 21a of the computer 21 and communicates them to the setup arm controller 41b, the robotic arm controller 41c, and the IDU controller 41d. The main cart controller 41a also manages instrument exchanges and the overall state of the movable cart 60, the robotic arm 40, and the IDU 52. The main cart controller 41a also communicates actual joint angles back to the controller 21a.

The setup arm controller 41b controls each of joints 63a and 63b, and the rotatable base 64 of the setup arm 62 and calculates desired motor movement commands (e.g., motor torque) for the pitch axis and controls the brakes. The robotic arm controller 41c controls each joint 44a and 44b of the robotic arm 40 and calculates desired motor torques required for gravity compensation, friction compensation, and closed loop position control of the robotic arm 40. The robotic arm controller 41c calculates a movement command based on the calculated torque. The calculated motor commands are then communicated to one or more of the actuators 48a and 48b in the robotic arm 40. The actual joint positions are then transmitted by the actuators 48a and 48b back to the robotic arm controller 41c.

The IDU controller 41d receives desired joint angles for the surgical instrument 50, such as wrist and jaw angles, and computes desired currents for the motors in the IDU 52. The IDU controller 41d calculates actual angles based on the motor positions and transmits the actual angles back to the main cart controller 41a.

The robotic arm 40 is controlled as follows. Initially, a pose of the handle controller controlling the robotic arm 40, e.g., the handle controller 38a, is transformed into a desired pose of the robotic arm 40 through a hand eye transform function executed by the controller 21a. The hand eye function, as well as other functions described herein, is/are embodied in software executable by the controller 21a or any other suitable controller described herein. The pose of one of the handle controller 38a may be embodied as a coordinate position and role-pitch-yaw ("RPY") orientation relative to a coordinate reference frame, which is fixed to the surgical console 30. The desired pose of the instrument 50 is relative to a fixed frame on the robotic arm 40. The pose of the handle controller 38a is then scaled by a scaling function executed by the controller 21a. In embodiments, the coordinate position is scaled down and the orientation is scaled up by the scaling function. In addition, the controller 21a also executes a clutching function, which disengages the handle controller 38a from the robotic arm 40. In particular, the controller 21a stops transmitting movement commands from the handle controller 38a to the robotic arm 40 if certain movement limits or other thresholds are exceeded and in essence acts like a virtual clutch mechanism, e.g., limits mechanical input from effecting mechanical output.

The desired pose of the robotic arm 40 is based on the pose of the handle controller 38a and is then passed by an inverse kinematics function executed by the controller 21a. The inverse kinematics function calculates angles for the joints 44a, 44b, 44c of the robotic arm 40 that achieve the scaled and adjusted pose input by the handle controller 38a. The calculated angles are then passed to the robotic arm controller 41c, which includes a joint axis controller having a proportional-derivative (PD) controller, the friction estimator module, the gravity compensator module, and a two-sided saturation block, which is configured to limit the commanded torque of the motors of the joints 44a, 44b, 44c.

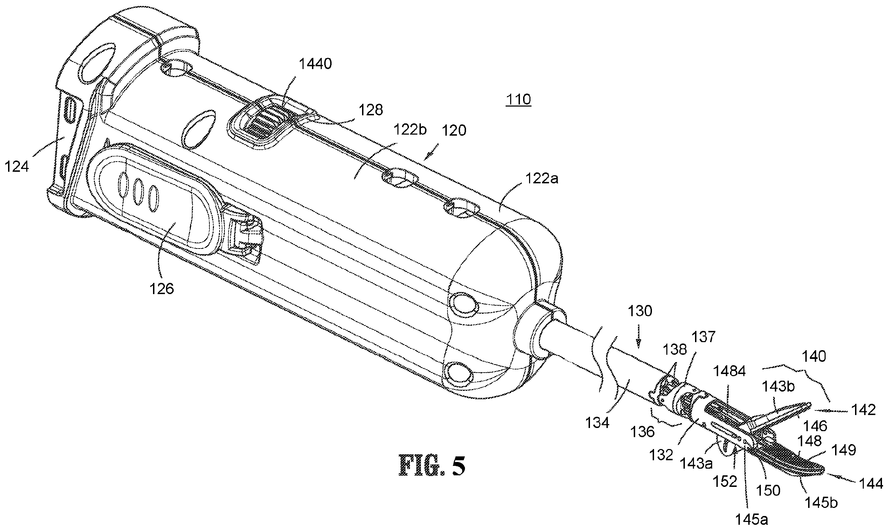

Turning to FIGS. 5-7, a surgical instrument 110 provided in accordance with the present disclosure generally includes a housing 120, a shaft 130 extending distally from housing 120, an end effector assembly 140 extending distally from shaft 130, and an actuation assembly 1100 disposed within housing 120 and operably associated with end effector assembly 140. Instrument 110 is detailed herein as an articulating electrosurgical forceps configured for use with a surgical robotic system, e.g., surgical robotic system 10 (FIG. 1). However, the aspects and features of instrument 110 provided in accordance with the present disclosure, detailed below, are equally applicable for use with other suitable surgical instruments, e.g., graspers, staplers, clip appliers, and/or in other suitable surgical systems, e.g., motorized, other power-driven systems, and/or manually-actuated surgical systems (including handheld instruments).

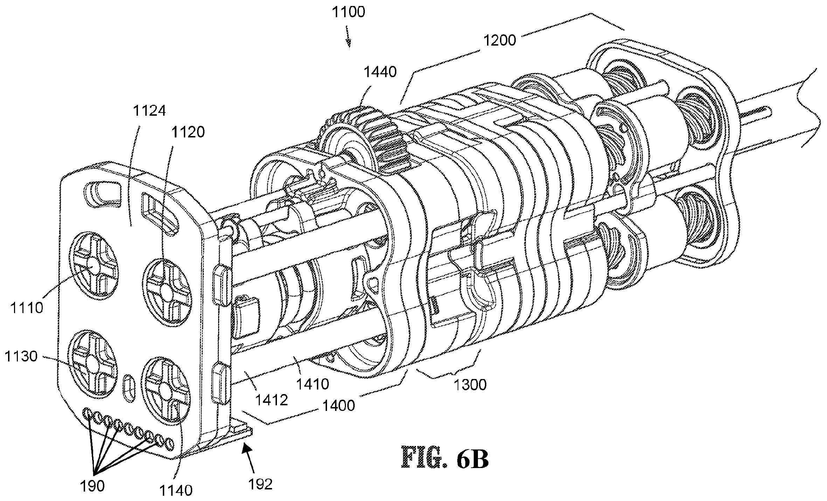

With particular reference to FIG. 5, housing 120 of instrument 110 includes first and second body portion 122a, 122b and a proximal face plate 124 that cooperate to enclose actuation assembly 1100 therein. Proximal face plate 124 includes apertures defined therein through which input couplers 1110-1140 (FIG. 6B) of actuation assembly 1100 extend. A pair of latch levers 126 (only one of which is illustrated in FIG. 5) extending outwardly from opposing sides of housing 120 enable releasable engagement of housing 120 with a robotic arm of a surgical robotic system, e.g., surgical robotic system 10 (FIG. 1). An aperture 128 defined through housing 120 permits thumbwheel 1440 to extend therethrough to enable manual manipulation of thumbwheel 1440 from the exterior of housing 120 to permit manual opening and closing of end effector assembly 140.

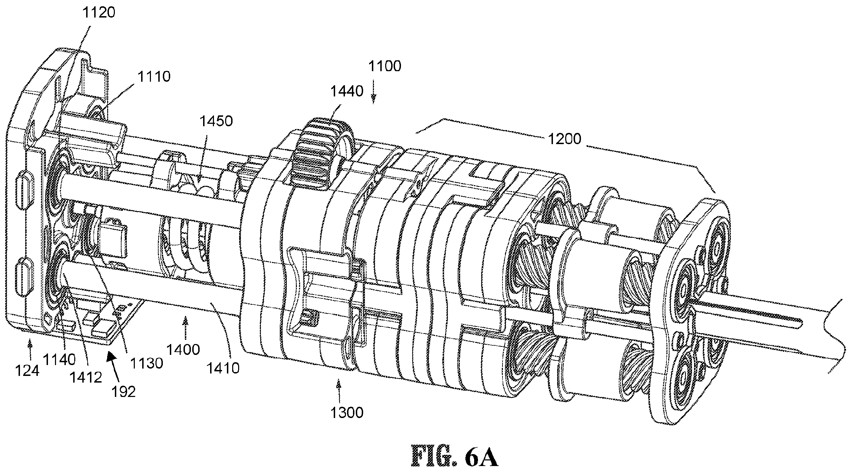

Referring also to FIGS. 6A-7, a plurality of electrical contacts 190 extend through one or more apertures defined through proximal face plate 124 to enable electrical communication between instrument 110 and surgical robotic system 10 (FIG. 1) when instrument 110 is engaged on a robotic arm thereof, e.g., for the communication of data, control, and/or power signals therebetween. As an alternative to electrical contacts 190 extending through proximal face plate 124, other suitable transmitter, receiver, and/or transceiver components to enable the communication of data, control, and/or power signals are also contemplated, e.g., using RFID, Bluetooth^{®}, WiFi^{®}, or via any other suitable wired, wireless, contacted, or contactless communication method. At least some of the electrical contacts 190 are electrically coupled with electronics 192 mounted on an interior side of proximal face plate 124, e.g., within housing 120. Electronics 192 may include, for example, a storage device, a communications device (including suitable input/output components), and a CPU including a memory and a processor. Electronics 192 may be mounted on a circuit board or otherwise configured, e.g., as a chip.

The storage device of electronics 192 stores information relating to surgical instrument such as, for example: the item number, e.g., SKU number; date of manufacture; manufacture location, e.g., location code; serial number; lot number; use information; setting information; adjustment information; calibration information; security information, e.g., encryption key(s), and/or other suitable additional or alternative data. The storage device of electronics 192 may be, for example, a magnetic disk, flash memory, optical disk, or other suitable data storage device.

As an alternative or in addition to storing the above-noted information in the storage device of electronics 192, some or all of such information, e.g., the use information, calibration information, setting information, and/or adjustment information, may be stored in a storage device associated with surgical robotic system 10 (FIG. 1), a remote server, a cloud server, etc., and accessible via instrument 110 and/or surgical robotic system 10 (FIG. 1). In such configurations, the information may, for example, be updated by manufacturer-provided updates, and/or may be applied to individual instruments, units of instruments (e.g., units from the same manufacturing location, manufacturing period, lot number, etc.), or across all instruments. Further still, even where the information is stored locally on each instrument, this information may be updated by manufacturer-provided updates manually or automatically upon connection to the surgical robotic system 10 (FIG. 1).

Referring again to FIG. 5, shaft 130 of instrument 110 includes a distal segment 132, a proximal segment 134, and an articulating section 136 disposed between the distal and proximal segments 132, 134, respectively. Articulating section 136 includes one or more articulating components 137, e.g., links, joints, etc. A plurality of articulation cables 138, e.g., four (4) articulation cables, or other suitable actuators, extend through articulating section 136. More specifically, articulation cables 138 are operably coupled to distal segment 132 of shaft 130 at the distal ends thereof and extend proximally from distal segment 132 of shaft 130, through articulating section 136 of shaft 130 and proximal segment 134 of shaft 130, and into housing 120, wherein articulation cables 138 operably couple with an articulation sub-assembly 1200 of actuation assembly 1100 to enable selective articulation of distal segment 132 (and, thus end effector assembly 140) relative to proximal segment 134 and housing 120, e.g., about at least two axes of articulation (yaw and pitch articulation, for example). Articulation cables 138 are arranged in a generally rectangular configuration, although other suitable configurations are also contemplated. In some configurations, as an alternative, shaft 130 is substantially rigid, malleable, or flexible and not configured for active articulation. Articulation sub-assembly 1200 is described in greater detail below.

With respect to articulation of end effector assembly 140 relative to proximal segment 134 of shaft 130, actuation of articulation cables 138 may be accomplished in pairs. More specifically, in order to pitch end effector assembly 140, the upper pair of cables 138 are actuated in a similar manner while the lower pair of cables 138 are actuated in a similar manner relative to one another but an opposite manner relative to the upper pair of cables 138. With respect to yaw articulation, the right pair of cables 138 are actuated in a similar manner while the left pair of cables 138 are actuated in a similar manner relative to one another but an opposite manner relative to the right pair of cables 138. Other configurations of articulation cables 138 or other articulation actuators are also contemplated.

Continuing with reference to FIG. 5, end effector assembly 140 includes first and second jaw members 142, 144, respectively. Each jaw member 142, 144 includes a proximal flange portion 143a, 145a and a distal body portion 143b, 145b, respectively. Distal body portions 143b, 145b define opposed tissue-contacting surfaces 146, 148, respectively. Proximal flange portions 143a, 145a are pivotably coupled to one another about a pivot 150 and are operably coupled to one another via a cam-slot assembly 152 including a cam pin slidably received within cam slots defined within the proximal flange portion 143a, 145a of at least one of the jaw members 142, 144, respectively, to enable pivoting of jaw member 142 relative to jaw member 144 and distal segment 132 of shaft 130 between a spaced-apart position (e.g., an open position of end effector assembly 140) and an approximated position (e.g., a closed position of end effector assembly 140) for grasping tissue between tissue-contacting surfaces 146, 148. As an alternative to this unilateral configuration, a bilateral configuration may be provided whereby both jaw members 142, 144 are pivotable relative to one another and distal segment 132 of shaft 130. Other suitable jaw actuation mechanisms are also contemplated.

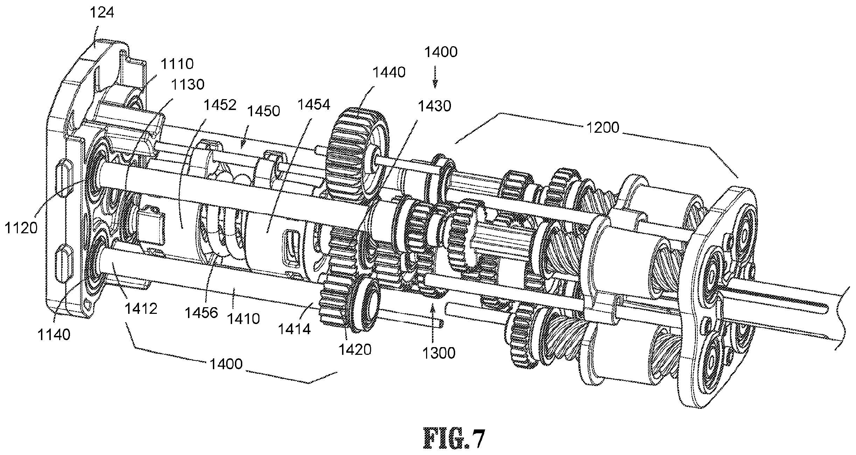

In configurations, a longitudinally-extending knife channel 149 (only knife channel 149 of jaw member 144 is illustrated; the knife channel of jaw member 142 is similarly configured) is defined through the tissue-contacting surface 146, 148 of one or both jaw members 142, 144. In such aspects, a knife assembly including a knife tube (not shown) extending from housing 120 through shaft 130 to end effector assembly 140 and a knife blade (not shown) disposed within end effector assembly 140 between jaw members 142, 144 is provided. The knife blade is selectively translatable through the knife channel(s) 149 and between the jaw member 142, 144 to cut tissue grasped between tissue-contacting surfaces 146, 148 of jaw members 142, 144, respectively. The knife tube is operably coupled to a knife drive sub-assembly 1300 (FIG. 7) of actuation assembly 1100 (FIGS. 6A-6B) at a proximal end thereof to enable the selective actuation of the knife tube to, in turn, reciprocate the knife blade (not shown) between jaw members 142, 144 to cut tissue grasped between tissue-contacting surfaces 146, 148. As an alternative to a longitudinally-advancable mechanical knife, other suitable mechanical cutters are also contemplated, e.g., guillotine-style cutters, as are energy-based cutters, e.g., RF electrical cutters, ultrasonic cutters, etc., in static or dynamic configurations.

Referring still to FIG. 5, a drive rod 1484 is operably coupled to cam-slot assembly 152 of end effector assembly 140, e.g., engaged with the cam pin thereof, such that longitudinal actuation of drive rod 1484 pivots jaw member 142 relative to jaw member 144 between the spaced-apart and approximated positions. More specifically, urging drive rod 1484 proximally pivots jaw member 142 relative to jaw member 144 towards the approximated position while urging drive rod 1484 distally pivots jaw member 142 relative to jaw member 144 towards the spaced-apart position. However, other suitable mechanisms and/or configurations for pivoting jaw member 142 relative to jaw member 144 between the spaced-apart and approximated positions in response to selective actuation of drive rod 1484 are also contemplated. Drive rod 1484 extends proximally from end effector assembly 140 through shaft 130 and into housing 120 wherein drive rod 1484 is operably coupled with a jaw drive sub-assembly 1400 of actuation assembly 1100 (FIGS. 6A-6B) to enable selective actuation of end effector assembly 140 to grasp tissue therebetween and apply a jaw force within an appropriate jaw force range.

Tissue-contacting surfaces 146, 148 of jaw members 142, 144, respectively, are at least partially formed from an electrically conductive material and are energizable to different potentials to enable the conduction of RF electrical energy through tissue grasped therebetween, although tissue-contacting surfaces 146, 148 may alternatively be configured to supply any suitable energy, e.g., thermal, microwave, light, ultrasonic, ultrasound, etc., through tissue grasped therebetween for energy-based tissue treatment. Instrument 110 defines a conductive pathway (not shown) through housing 120 and shaft 130 to end effector assembly 140 that may include lead wires, contacts, and/or electrically-conductive components to enable electrical connection of tissue-contacting surfaces 146, 148 of jaw members 142, 144, respectively, to an energy source (not shown), e.g., an electrosurgical generator, for supplying energy to tissue-contacting surfaces 146, 148 to treat, e.g., seal, tissue grasped between tissue-contacting surfaces 146, 148.

With additional reference to FIGS. 6A-7, as noted above, actuation assembly 1100 is disposed within housing 120 and includes an articulation sub-assembly 1200, a knife drive sub-assembly 1300, and a jaw drive sub-assembly 1400. Articulation sub-assembly 1200 is operably coupled between first and second input couplers 1110, 1120, respectively, of actuation assembly 1100 and articulation cables 138 (FIG. 5) such that, upon receipt of appropriate inputs into first and/or second input couplers 1110, 1120, articulation sub-assembly 1200 manipulates cables 138 (FIG. 5) to articulate end effector assembly 140 in a desired direction, e.g., to pitch and/or yaw end effector assembly 140. Articulation sub-assembly 1200 is described in greater detail below.

Knife drive sub-assembly 1300 is operably coupled between third input coupler 1130 of actuation assembly 1100 and the knife tube such that, upon receipt of appropriate input into third input coupler 1130, knife drive sub-assembly 1300 manipulates the knife tube to reciprocate the knife blade between jaw members 142, 144 to cut tissue grasped between tissue-contacting surfaces 146, 148.

Jaw drive sub-assembly 1400 is operably coupled between fourth input coupler 1140 of actuation assembly 1100 and drive rod 1484 such that, upon receipt of appropriate input into fourth input coupler 1140, jaw drive sub-assembly 1400 pivots jaw members 142, 144 between the spaced-apart and approximated positions to grasp tissue therebetween and apply a jaw force within an appropriate jaw force range.

Actuation assembly 1100 is configured to operably interface with a surgical robotic system, e.g., system 10 (FIG. 1), when instrument 110 is mounted on a robotic arm thereof, to enable robotic operation of actuation assembly 1100 to provide the above-detailed functionality. That is, surgical robotic system 10 (FIG. 1) selectively provides inputs, e.g., rotational inputs to input couplers 1110-140 of actuation assembly 1100 to articulate end effector assembly 140, grasp tissue between jaw members 142, 144, and/or cut tissue grasped between jaw members 142, 144. However, as noted above, it is also contemplated that actuation assembly 1100 be configured to interface with any other suitable surgical systems, e.g., a manual surgical handle, a powered surgical handle, etc.

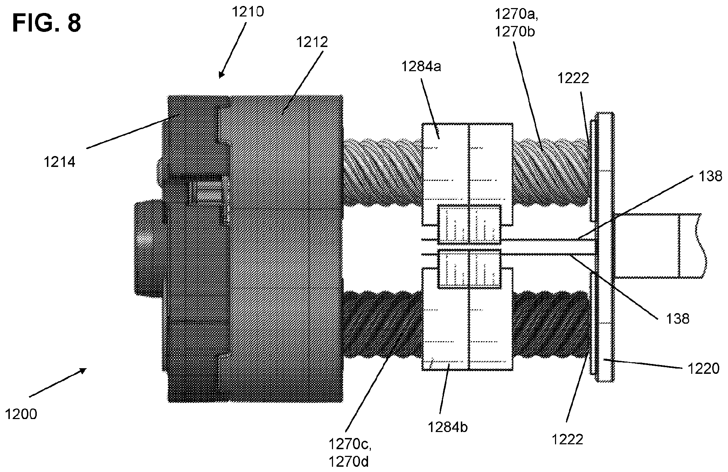

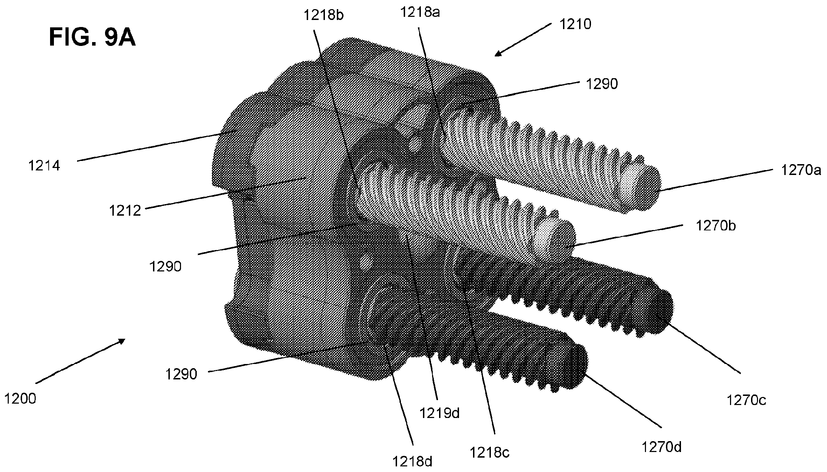

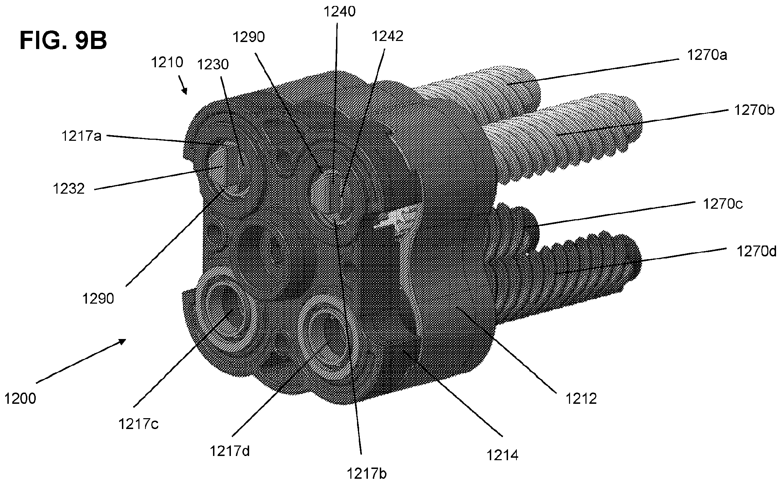

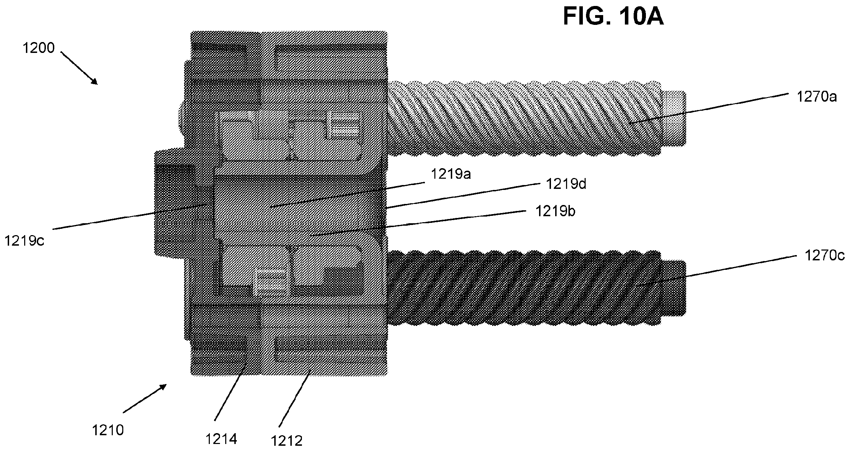

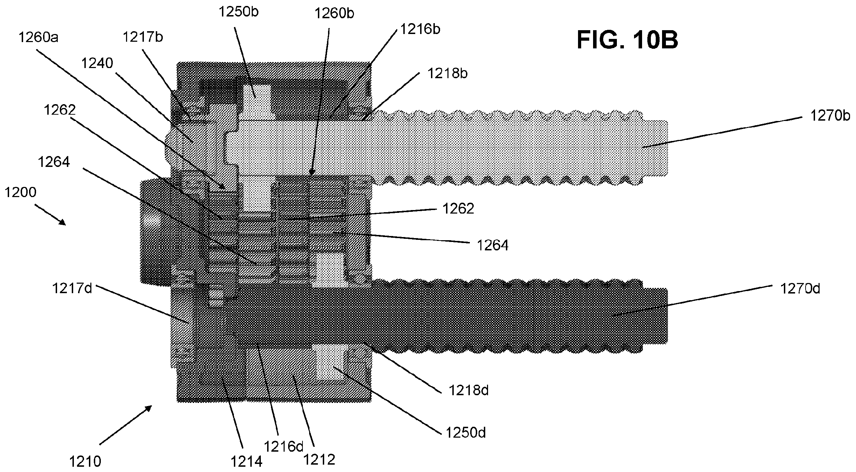

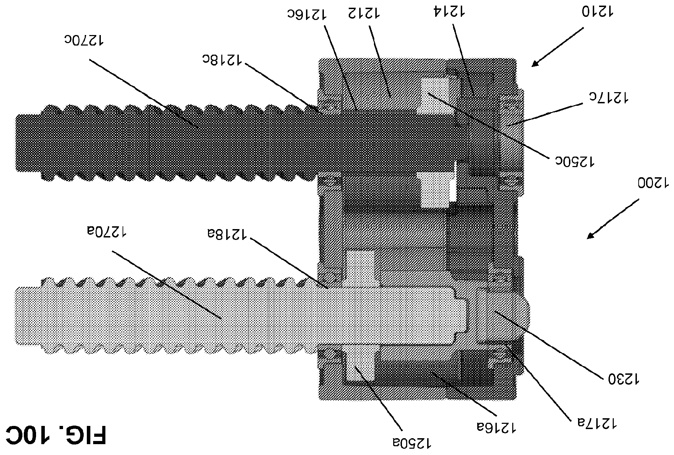

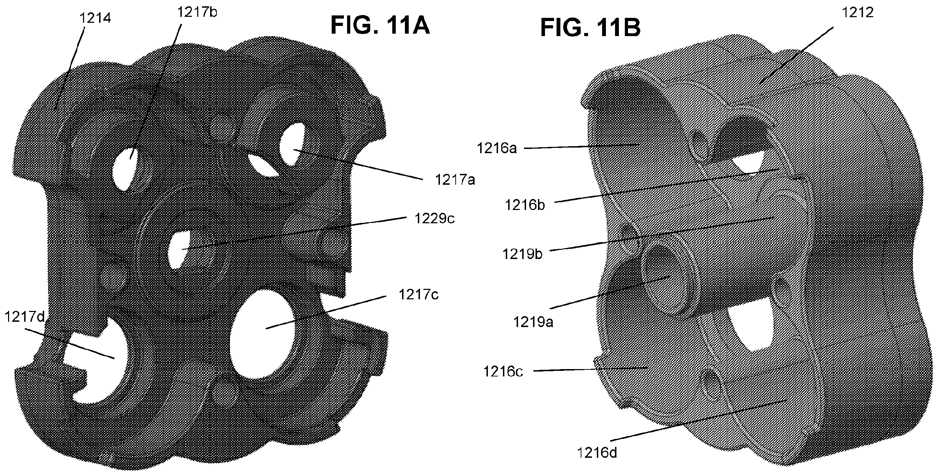

Turning to FIGS. 8-16B, articulation sub-assembly 1200, as noted above, is operably coupled between first and second input couplers 1110, 1120, respectively, of actuation assembly 1100 and articulation cables 138 (FIG. 5) such that, upon receipt of appropriate inputs into first and/or second input couplers 1110, 1120, articulation sub-assembly 1200 manipulates cables 138 (FIG. 5) to articulate end effector assembly 140 in a desired direction, e.g., to pitch and/or yaw end effector assembly 140. Articulation sub-assembly 1200, more specifically, includes a proximal housing 1210, a distal support plate 1220, two input shafts 1230, 1240, four output gears 1250a-d, proximal and distal coupling gears 1260a, 1260b, and four lead screws 1270a-d.

Referring to FIGS. 8-11B, proximal housing 1210 is formed from a housing base 1212 and a housing cap 1214 that are configured to engage one another in any suitable manner, e.g., via snap-fitting or other suitable mechanical latching engagement, via interference fitting, via adhesives, via overmolding, etc. Housing base 1212 and housing cap 1214 may be releasably engaged to enable disassembly at the end user (or re-processor) or may be configured for permanent engagement after manufacturing.

Proximal housing 1210 defines four internal cavities 1216a-d extending longitudinally through proximal housing 1210 and generally arranged in a rectangular (e.g., square) configuration with each cavity 1216a-d positioned towards one of the four corners of proximal housing 1210. Proximal and distal apertures 1217a-d and 1218a-d, respectively, establish access to cavities 1216a-d through the proximal and distal faces, respectively, of proximal housing 1210 defined by housing cap 1214 and housing base 1212, respectively. Each cavity 1216a-1216d is configured to capture a corresponding output gear 1250a-d and corresponding lead screw 1270a-d therein while permitting rotation of the captured output gear 1250a-d and corresponding lead screw 1270a-d within proximal housing 1210. Further, two of the cavities 1216a, 1216b each partially house one of the two input shafts 1230, 1240 therein proximally of the corresponding output gear 1250a, 1250b housed therein. Proximal receivers 1232, 1242 of input shafts 1230, 1240, respectively, extend from cavities 1216a, 1216b at least partially through proximal apertures 1217a, 1217b to enable input shafts 1230, 1240 to receive external rotational inputs proximally of proximal housing 1210.

Proximal housing 1210 further includes a longitudinally-extending central lumen 1219a defined by a cylindrical body 1219b extending proximally from the interior of housing base 1212 to housing cap 1214. Proximal and distal apertures 1219c, 1219d, respectively, establish access to central lumen 1219a through the proximal and distal faces, respectively, of proximal housing 1210 defined by housing cap 1214 and housing base 1212, respectively. Proximal and distal coupling gears 1260a, 1260b are rotatably disposed about cylindrical body 1219b and are operably retained within proximal housing 1210 such that: proximal coupling gear 1260a operably couples input shaft 1240 with output gears 1250b, 12050d such that a rotational input provided to input shaft 1240 rotates output gears 1250b, 1250d in the same direction with equal magnitude; and such that distal coupling gear 1260b operably couples input shaft 1230 with output gears 1250a, 1250c such that a rotational input provided to input shaft 1230 rotates output gears 1250a, 1250c in the same direction with equal magnitude.

Proximal end portions of lead screws 1270a-d extend proximally through distal apertures 1218a-d of proximal housing 1210, respectively, to operably support respective output gears 1250a-d thereon in fixed rotational orientation such that rotation of output gears 1250a-d rotate the corresponding lead screws 1270a-d. In aspects, bearings 1290 are disposed within distal apertures 1218a-d to minimize friction and facilitate rotation of lead screws 1270a-d relative to proximal housing 1210. Likewise, bearings 1290 may be disposed within proximal apertures 1217a, 1217b to minimize friction and facilitate rotation of input shafts 1230, 1240, respectively, relative to proximal housing 1210.

Distal support plate 1220 rotatably supports distal dock ends 1282 of lead screws 1270a-1270d, respectively, within apertures 1222 thereof. Distal support plate 1220 is distally-spaced from proximal housing 1210. Distal support plate 1220 and/or proximal housing 1210 may further support one or more anti-rotation bars (not explicitly shown) extending therebetween. The anti-rotation bars operably couple with collars 1284a, 1284b of lead screws 1270a-1270d to inhibit rotation of collars 1284a, 1284b thereby confining collars 1284a, 1284b to translational motion in response to rotation of the lead screws 1270a-d.

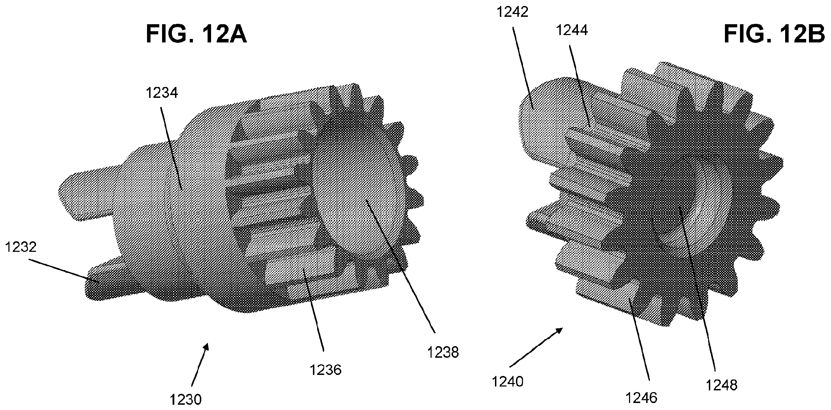

With reference to FIGS. 12A-12B, in conjunction with FIGS. 8-10C, input shafts 1230, 1240, as noted above, are housed within cavities 1216a, 1216b of proximal housing 1210 proximally of output gears 1250a, 1250b. Input shafts 1230a, 1230b, more specifically, each include a proximal receiver 1232, 1242 configured to couple to input couplers 1110, 1120 (FIG. 6B), e.g., via coupling shafts, in fixed rotational orientation relative thereto such that rotational inputs provided to input couplers 1110, 1120 (FIG. 6B) are transmitted to input shafts 1230, 1240 to thereby rotate input shafts 1230, 1240 in the same direction and with the same magnitude. Alternatively, suitable gearing may be provided to amplify or attenuate the magnitude of the rotational input provided to input shafts 1230, 1240 as compared to the rotational input provided to input couplers 1110, 1120 and/or reversing gears may be provided to reverse the direction of rotation of either or both input shafts 1230, 1240 as compared to the rotational inputs provided to the corresponding input couplers 1110, 1120,

Input shafts 1230, 1240 further include body portions 1234, 1244 extending distally from proximal receivers 1232, 1234 and distal gears 1236, 1246 formed at the distal end portions of respective body portions 1234, 1244. Body portion 1234 of input shaft 1230 defines a length greater than a length of body portion 1244 of input shaft 1240 such that distal gear 1236 of input shaft 1230 is offset distally within proximal housing 1210 and relative to distal gear 1246 of input shaft 1240, and such that distal gear 1246 of input shaft 1240 is offset proximally within proximal housing 1210 and relative to distal gear 136 of input shaft 1230. Body portion 1234 of input shaft 1230 defines an elongated internal recess 1238 configured to receive proximal dock end 1274 of lead screw 1270a while body portion 1244 of input shaft 1240 defines a shallow recess 1248 configured to receive proximal dock end 1274 of lead screw 1270b.

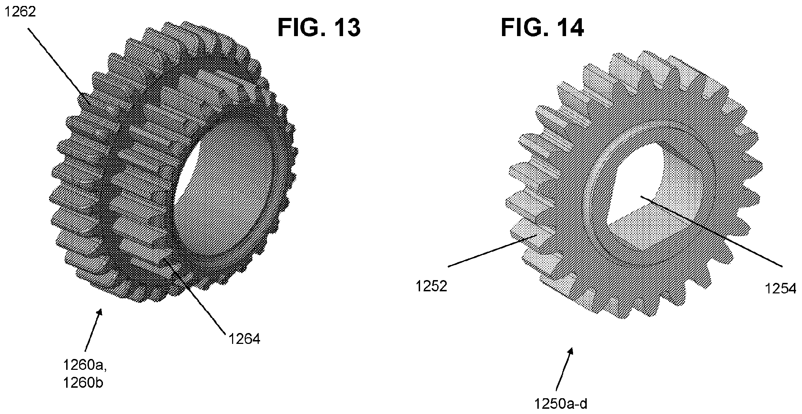

Referring to FIG. 13, in conjunction with FIGS. 8-10C, proximal and distal coupling gears 1260a, 1260b are compound gears each including a major gear 1262 and a minor gear 1264, are coaxially aligned with one another, and are longitudinally offset with respect to one another. This longitudinal offset substantially matches the longitudinal offset of distal gears 1236, 1246 of input shafts 1230, 1240, such that major gear 1262 of proximal coupling gear 1260a is disposed in meshed engagement with distal gear 1246 of input shaft 1240 while major gear 1262 of distal coupling gear 1260b is disposed in meshed engagement with distal gear 1236 of input shaft 1230, all within proximal housing 1210.

With reference to FIG. 14, in conjunction with FIGS. 8-10C, each output gear 1250a-d defines external annular gear teeth 1252 and an aperture 1254 extending therethrough. Apertures 1254 define non-circular geometric shapes such as, for example, straight-segment polygons, curved-segment polygons, combinations of curved and straight segment polygons, etc. Other suitable non-circular geometric shapes, including irregular and asymmetrical shapes are also contemplated. External annular gear teeth 1252 of output gears 1250b, 1250d are disposed in meshed engagement with minor gear 1264 of proximal coupling gear 1260a on diagonally-opposed sides thereof, while external annular gear teeth 1252 of output gears 1250a, 1250c are disposed in meshed engagement with minor gear 1264 of distal coupling gear 1260b on diagonally-opposed sides thereof. As a result of the length difference between input shafts 1230, 1240, and the offset configuration of coupling gears 1260a, 1260b, output gears 1250a, 1250c are transversely aligned relative to one another but positioned more-distally within proximal housing 1210 as compared to output gears 1250b, 1250d, while output gears 1250b, 1250d are transversely aligned relative to one another but positioned more-proximally within proximal housing 1210 as compared to output gears 1250a, 1250c.

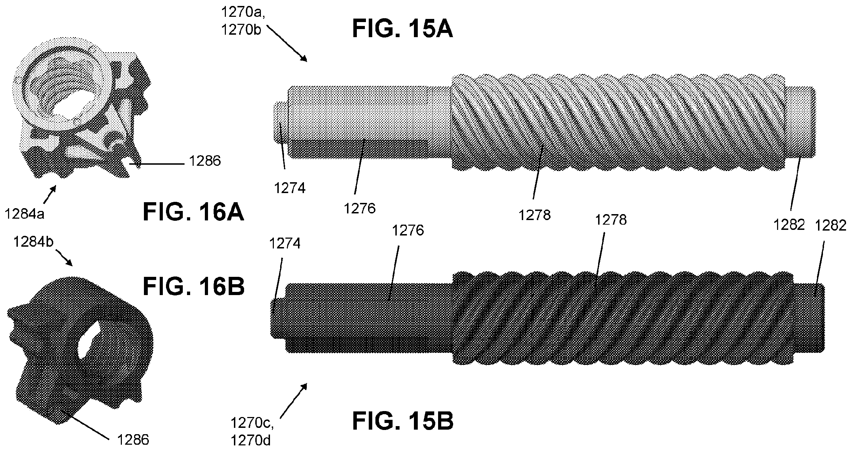

Turning to FIGS. 15A-16B, in conjunction with FIGS. 8-10C, lead screw 1270a, 1270b are identical to one another and lead screws 1270c, 1270d are identical to one another. Collars 1284a are configured for use with lead screws 1270a, 1270b, while collars 1284b are configured for use with lead screws 1270c, 1270d. Each lead screw 1270a-d includes a proximal dock portion 1274, a non-cylindrical proximal shaft portion 1276, a threaded distal portion 1278, and a distal dock portion 1282. A collar 1284a, 1284b is operably threadingly engaged about the threaded distal portion 1278 of the corresponding lead screw 1270a-d such that rotation of the lead screw 1270a-d translates the corresponding collar 1284a, 1284b longitudinally therealong. Collars 1284a, 1284b are configured to secure proximal end portions of articulation cables 138 (FIG. 5), e.g., within ferrules 1286 thereof, such that proximal translation of a collar 1284a, 1284b tensions the corresponding articulation cable 138 (FIG. 5) while distal translation of a collar 1284a, 1284b de-tensions the corresponding articulation cable 138 (FIG. 5).

Threaded distal portions 1278 of lead screws 1270a-d are at least partially exposed between proximal housing 1210 and distal base plate 1220 and define threading having pitches of equal magnitude; however, threaded distal portions 1278 of lead screws 1270a, 1270b are pitched in an opposite direction relative to threaded distal portions 1278 of lead screws 1270c, 1270d. Thus, in response to rotation of lead screws 1270a, 1270c in the same direction and with equal magnitude, the collars 1284a, 1284b thereof (which are configured to threadingly engage the corresponding threading of lead screws, 1270a, 1270c) are translated with equal magnitude in opposite directions. Likewise, in response to rotation of lead screws 1270b, 1270d in the same direction and with equal magnitude, the collars 1284a, 1284b thereof (which are configured to threadingly engage the corresponding threading of lead screws, 1270b, 1270d) are translated with equal magnitude in opposite directions. Distal dock portions 1282 of lead screws 1270a-d are rotationally seated within apertures 1222 defined within distal support plate 1220.

Proximal dock portions 1274 and non-cylindrical proximal shaft portions 1276 of lead screws 1270a-d extend proximally through distal apertures 1218a-d, respectively, and into proximal housing 1210 whereby proximal dock portion 1274 of lead screw 1270a is rotationally seated within elongated internal recess 1238 of body portion 1234 of input shaft 1230, proximal dock portion 1274 of lead screw 1270b is rotationally seated within shallow internal recess 1248 of body portion 1244 of input shaft 1240, and proximal dock portions 1274 of lead screws 1270c, 1270d are rotationally seated within proximal apertures 1217c, 1217d, respectively, of proximal housing 1210.

Non-cylindrical proximal shaft portions 1276 of lead screws 1270a-d extend partially through proximal housing 1210 and through apertures 1254 of output gears 1250-a-d, respectively. Non-cylindrical proximal shaft portions 1276 define cross-sectional shapes, e.g., non-circular geometric shapes such as, for example, straight-segment polygons, curved-segment polygons, combinations of curved and straight segment polygons, other suitable non-circular geometric shapes including irregular and asymmetrical shapes, etc., that are at least partially complementary to apertures 1254 such that output gears 1250a-d are rotationally fixed about non-cylindrical proximal shaft portions 1276 of lead screws 1270a-d within proximal housing 1210. In this manner, rotation of an output gear 1250a-d effects like rotation of the corresponding lead screw 1270a-1270d.

Referring generally to FIGS. 8-10C, as a result of the above-detailed configuration, a rotational input provided to input coupler 1110 (FIG. 6B) drives rotation of input shaft 1230 which, in turn, drives rotation of distal coupling gear 1260b. Rotation of distal coupling gear 1260b drives equal rotation of output gears 1250a, 1250c due to the meshed engagement therewith, to thereby rotate lead screws 1270a, 1270c in the same direction with equal magnitude. Since threaded distal portions 1278 of lead screws 1270a, 120c are pitched in opposite directions, the similar direction and magnitude of rotation imparted to lead screws 1270a, 1270c results in equal magnitude and opposite direction translation of collars 1284a, 1284b. Collars 1284a, 1284b are configured to securely engage proximal end portions of a diagonally-opposed pair of first and third articulation cables 138 (FIG. 5). Thus, proximal translation of a collar 1284a pulls and tensions the first articulation cable 138 (FIG. 5) while distal translation of collar 1284b pushes or de-tensions the third articulation cable 138 (FIG. 5). Accordingly, a first rotational input provided to input coupler 1110 tensions the first articulation cable 138 (FIG. 5) and de-tensions the third, diagonally-opposed articulation cable 138 (FIG. 5). A second, opposite rotational input provided to input coupler 1110, on the other hand de-tensions the first articulation cable 138 (FIG. 5) and tensions the third, diagonally-opposed articulation cable 138 (FIG. 5).

Similarly as above, a rotational input provided to input coupler 1120 drives rotation of input shaft 1240 which, in turn, drives rotation of proximal coupling gear 1260a. Rotation of proximal coupling gear 1260a drives equal rotation of output gears 1250b, 1250d due to the meshed engagement therewith, to thereby rotate lead screws 1270b, 1270d in the same direction with equal magnitude. Since threaded distal portions 1278 of lead screws 1270b, 1270d are pitched in opposite directions, the similar direction and magnitude of rotation imparted to lead screws 1270b, 1270d results in equal magnitude and opposite direction translation of collars 1284a, 1284b. Collars 1284a, 1284b are configured to securely engage proximal end portions of a diagonally-opposed pair of second and fourth articulation cables 138 (FIG. 5). Thus, proximal translation of a collar 1284a pulls and tensions the second articulation cable 138 (FIG. 5) while distal translation of a collar 1284b pushes or de-tensions the fourth articulation cable 138 (FIG. 5). Accordingly, a first rotational input provided to input coupler 1120 tensions the second articulation cable 138 (FIG. 5) and de-tensions the fourth, diagonally-opposed articulation cable 138 (FIG. 5). A second, opposite rotational input provided to input coupler 1120, on the other hand de-tensions the second articulation cable 138 (FIG. 5) and tensions the fourth, diagonally-opposed articulation cable 138 (FIG. 5).

Continuing with reference to FIGS. 8-10C, and with additional reference to FIG. 5, articulation sub-assembly 1200, as detailed above, enables: upward pitch articulation of end effector assembly 140, e.g., wherein equal magnitude and first direction rotational inputs are provided to input couplers 1110, 1120; downward pitch articulation of end effector assembly 140, e.g., wherein equal magnitude and second, opposite direction rotational inputs are provided to input couplers 1110, 1120; right yaw articulation of end effector assembly 140, e.g., wherein equal magnitude rotational inputs are provided to input couplers 1110, 1120 with the first direction of rotation imparted to input coupler 1110 and the second direction of rotation imparted to input coupler 1110; and left yaw articulation of end effector assembly 140, e.g., wherein equal magnitude rotational inputs are provided to input couplers 1110, 1120 with the second direction of rotation imparted to input coupler 1110 and the second direction of rotation imparted to input coupler 1120. Combinations of pitch and/or yaw articulation of end effector assembly 140 may be achieved by varying the rotational inputs provided to input couplers 1110, 1120 to provide a desired orientation of end effector assembly 140 (FIG. 5).

The above-detailed components of articulation sub-assembly 1200 are formed from durable, sterilizable materials to enable use of articulation sub-assembly 1220 in multiple different surgical procedures with sterilization and/or cleaning processes between each use (with or without dis-assembly of proximal housing 1210). Further, the configuration of articulation sub-assembly 1200 provides suitable access to the components of articulation sub-assembly 1200 to enable sufficient sterilization and/or cleaning (as required for such reusable surgical instruments) without or with minimal disassembly, thus further facilitating re-use.

The above-detailed articulation sub-assembly 1200, although described with respect to an articulating electrosurgical forceps, may alternatively be utilized in any other suitable articulating instrument. Further, as articulation sub-assembly 1200 is substantially self-contained, articulation sub-assembly 1200 may be utilized as part of a modular system where articulation sub-assembly 1200 is "dropped-in" to a desired instrument to provide articulation functionality thereto.

It should be understood that various aspects disclosed herein may be combined in different combinations than the combinations specifically presented hereinabove and in the accompanying drawings. **In** addition, while certain aspects of the present disclosure are described as being performed by a single module or unit for purposes of clarity, it should be understood that the techniques of this disclosure may be performed by a combination of units or modules associated with, for example, a surgical system.

While several aspects of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular aspects. The invention is defined by the following claims.

## Claims

1. An articulation assembly for a surgical instrument, comprising:
a proximal housing (1210) including a central cylindrical body (1219b) disposed therein and first, second, third, and fourth internal cavities (1216 a-d) defined therein and arranged about the central cylindrical body;
first and second coupling gears (1260a, 1260b) coaxially disposed about the central cylindrical body within the proximal housing, wherein the first coupling gear is disposed more-distally and the second coupling gear is disposed more-proximally;
first and second input shafts (1230, 1240) disposed at least partially within the first and second cavities, respectively, the first and second input shafts including proximal receivers (1232, 1242) extending proximally from the proximal housing and configured to receive first and second rotational inputs, the first and second input shafts including respective first and second gears (1236, 1246) wherein the first gear is disposed more-distally and in meshed engagement with the first coupling gear and the second gear is disposed more-proximally and in meshed engagement with the second coupling gear such that the first rotational input rotates the first input shaft to thereby rotate the first coupling gear and such that the second rotational input rotates the second input shaft to thereby rotate the second coupling gear; and
first, second, third, and fourth output gears (1250 a-d) disposed within the proximal housing, the first output gear (1250a) disposed within the first cavity (1216a) distally of the first input shaft and in meshed engagement with the first coupling gear, the second output gear (1250b) disposed within the second cavity (1216b) distally of the second input shaft and in meshed engagement with the second coupling gear, the third output gear (1250c) disposed within the third cavity (1216c) and in meshed engagement with the first coupling gear, and the fourth output gear (1250d) disposed within the fourth cavity (1216d) and in meshed engagement with the second coupling gear, wherein the first rotational input rotates the first and third output gears in the same direction with equal magnitude and wherein the second rotational input rotates the second and fourth output gears in the same direction with equal magnitude.

2. The articulation assembly according to claim 1, wherein the first and third output gears are diagonally opposite one another and wherein the second and fourth output gears are diagonally opposite one another.

3. The articulation assembly according to claim 1, wherein the first and second coupling gears are compound gears each including a major gear (1262) and a minor gear (1264).

4. The articulation assembly according to claim 3, wherein one of the major gears or the minor gears of the first and second coupling gears are disposed in meshed engagement with the first and second gears of the first and second input shafts, respectively.

5. The articulation assembly according to claim 4, wherein the other of the major gears or the minor gears of the first and second coupling gears are disposed in meshed engagement with the first and third output gears and the second and fourth output gears, respectively.

6. The articulation assembly according to claim 1, further comprising first, second, third, and fourth lead screws (1270a-d) extending proximally into the first, second, third, and fourth cavities and rotatably fixed relative to the first, second, third, and fourth output gears within the proximal housing such that rotation of one of the first, second, third, or fourth output gears rotates a corresponding one of the first, second, third, or fourth lead screws.

7. The articulation assembly according to claim 6, further comprising first, second, third, and fourth collars (1284 a-d) threadingly engaged about the first, second, third, and fourth lead screws, respectively, such that rotation of one of the first, second, third, or fourth lead screws translates a corresponding one of the first, second, third, or fourth collars therealong.

8. The articulation assembly according to claim 7, wherein first, second, third, and fourth articulation cables (138) are connected to the first, second, third, and fourth collars, respectively, such that translation of one of the first, second, third, or fourth collars tensions or de-tensions a corresponding one of the first, second, third, or fourth articulation cables.

9. The articulation assembly according to claim 6, wherein a proximal shaft portion (1276) of each of the first, second, third, and fourth lead screws extends proximally into a corresponding one of the first, second, third, or fourth cavities, and supports a corresponding one of the first, second, third, or fourth output gears thereon within the proximal housing.

10. The articulation assembly according to claim 9, wherein the proximal shaft portion of each of the first, second, third, and fourth lead screws defines a non-cylindrical configuration and wherein each of the first, second, third, and fourth output gears defines an aperture (1217) shaped at least partially complementary to the proximal shaft portion of a corresponding one of the first, second, third, and fourth lead screws to thereby rotatably fix the first, second, third, and fourth output gears about the first, second, third, and fourth lead screws, respectively.

11. A surgical instrument (50), comprising:
a housing (120);
a shaft (130) extending distally from the housing, the shaft including an articulating section (1200);
an end effector assembly (140) extending distally from the shaft;
first, second, third, and fourth articulation cables (138) operably coupled to the articulating section and extending proximally through the shaft into the housing to proximal end portions thereof; and
the articulation assembly according to claim 1, wherein the proximal end portions of the first, second, third, and fourth articulation cables are operably coupled to the first, second, third, and fourth output gears, respectively.

## Patentansprüche

1. Gelenkanordnung für ein chirurgisches Instrument, die umfasst:
ein proximales Gehäuse (1210), das einen darin angeordneten zentralen zylindrischen Körper (1219b) und erste, zweite, dritte und vierte innere Hohlräume (1216 a-d) einschließt, die darin definiert und um den zentralen zylindrischen Körper angeordnet sind;
erste und zweite Kupplungszahnräder (1260a, 1260b), die koaxial um den zentralen zylindrischen Körper innerhalb des proximalen Gehäuses angeordnet sind, wobei das erste Kupplungszahnrad weiter distal angeordnet ist und das zweite Kupplungszahnrad weiter proximal angeordnet ist;
erste und zweite Eingangswellen (1230, 1240), die mindestens teilweise innerhalb des ersten bzw. zweiten Hohlraums angeordnet sind, wobei die ersten und zweiten Eingangswellen proximale Aufnahmen (1232, 1242) einschließen, die sich proximal von dem proximalen Gehäuse erstrecken und konfiguriert sind, um erste und zweite Dreheingaben aufzunehmen, wobei die ersten und zweiten Eingangswellen ein jeweiliges erstes und zweites Zahnrad (1236, 1246) einschließen, wobei das erste Zahnrad weiter distal angeordnet ist und in ineinandergreifendem Eingriff mit dem ersten Kupplungszahnrad steht und das zweite Zahnrad weiter proximal angeordnet ist und in ineinandergreifendem Eingriff mit dem zweiten Kupplungszahnrad steht, sodass die erste Dreheingabe die erste Eingangswelle dreht, um dadurch das erste Kupplungszahnrad zu drehen, und sodass die zweite Dreheingabe die zweite Eingangswelle dreht, um dadurch das zweite Kupplungszahnrad zu drehen; und
erste, zweite, dritte und vierte Abtriebszahnräder (1250 a-d), die innerhalb des proximalen Gehäuses angeordnet sind, wobei das erste Abtriebszahnrad (1250a) innerhalb des ersten Hohlraums (1216a) distal von der ersten Antriebswelle und in ineinandergreifendem Eingriff mit dem ersten Kupplungszahnrad angeordnet ist, das zweite Abtriebszahnrad (1250b) innerhalb des zweiten Hohlraums (1216b) distal von der zweiten Antriebswelle und in ineinandergreifendem Eingriff mit dem zweiten Kupplungszahnrad angeordnet ist, das dritte Abtriebszahnrad (1250c) innerhalb des dritten Hohlraums (1216c) und in ineinandergreifendem Eingriff mit dem ersten Kupplungszahnrad angeordnet ist, und das vierte Abtriebszahnrad (1250d) innerhalb des vierten Hohlraums (1216d) und in ineinandergreifendem Eingriff mit dem zweiten Kupplungszahnrad angeordnet ist, wobei die erste Dreheingabe die ersten und dritten Abtriebszahnräder in der gleichen Richtung mit gleicher Größe dreht und wobei die zweite Dreheingabe die zweiten und vierten Abtriebsräder in der gleichen Richtung mit gleicher Größe dreht.

2. Gelenkanordnung nach Anspruch 1, wobei die ersten und dritten Abtriebsräder einander diagonal gegenüberliegen und wobei die zweiten und vierten Abtriebsräder einander diagonal gegenüberliegen.

3. Gelenkanordnung nach Anspruch 1, wobei die ersten und zweiten Kupplungszahnräder Verbundzahnräder sind, die jeweils ein Hauptzahnrad (1262) und ein Nebenzahnrad (1264) einschließen.

4. Gelenkanordnung nach Anspruch 3, wobei eines der Hauptzahnräder oder der Nebenzahnräder der ersten und zweiten Kupplungszahnräder jeweils in ineinandergreifendem Eingriff mit den ersten und zweiten Zahnrädern der ersten und zweiten Eingangswellen angeordnet ist.

5. Gelenkanordnung nach Anspruch 4, wobei das andere der Hauptzahnräder oder der Nebenzahnräder der ersten und zweiten Kupplungszahnräder in ineinandergreifendem Eingriff mit den ersten und dritten Ausgangszahnrädern beziehungsweise den zweiten und vierten Ausgangszahnrädern angeordnet sind.

6. Gelenkanordnung nach Anspruch 1, ferner umfassend erste, zweite, dritte und vierte Gewindespindeln (1270a-d), die sich proximal in die ersten, zweiten, dritten und vierten Hohlräume erstrecken und relativ zu den ersten, zweiten, dritten und vierten Abtriebszahnrädern innerhalb des proximalen Gehäuses drehfest sind, sodass eine Drehung eines der ersten, zweiten, dritten oder vierten Abtriebszahnräder eine entsprechende der ersten, zweiten, dritten oder vierten Gewindespindeln dreht.

7. Gelenkanordnung nach Anspruch 6, ferner umfassend erste, zweite, dritte und vierte Kragen (1284 a-d), die jeweils über ein Gewinde um die ersten, zweiten, dritten und vierten Gewindespindeln in Eingriff stehen, sodass eine Drehung einer der ersten, zweiten, dritten oder vierten Gewindespindeln einen entsprechenden der ersten, zweiten, dritten oder vierten Kragen daran entlang verschiebt.

8. Gelenkanordnung nach Anspruch 7, wobei erste, zweite, dritte und vierte Gelenkkabel (138) jeweils mit den ersten, zweiten, dritten und vierten Kragen verbunden sind, sodass eine Verschiebung eines der ersten, zweiten, dritten oder vierten Kragen ein entsprechendes der ersten, zweiten, dritten oder vierten Gelenkkabel spannt oder entspannt.

9. Gelenkanordnung nach Anspruch 6, wobei sich ein proximaler Wellenabschnitt (1276) jeder der ersten, zweiten, dritten und vierten Gewindespindeln proximal in einen entsprechenden der ersten, zweiten, dritten oder vierten Hohlräume erstreckt und ein entsprechendes der ersten, zweiten, dritten oder vierten Abtriebszahnräder daran innerhalb des proximalen Gehäuses trägt.

10. Gelenkanordnung nach Anspruch 9, wobei der proximale Wellenabschnitt jeder der ersten, zweiten, dritten und vierten Gewindespindeln eine nichtzylindrische Konfiguration definiert und wobei jedes der ersten, zweiten, dritten und vierten Abtriebszahnräder eine Öffnung (1217) definiert, die mindestens teilweise komplementär zu dem proximalen Wellenabschnitt einer entsprechenden der ersten, zweiten, dritten und vierten Gewindespindeln geformt ist, um dadurch die ersten, zweiten, dritten und vierten Abtriebszahnräder jeweils drehfest um die ersten, zweiten, dritten und vierten Gewindespindeln zu fixieren.

11. Chirurgisches Instrument (50), umfassend:
ein Gehäuse (120);
eine Welle (130), die sich von dem Gehäuse distal erstreckt, wobei die Welle einen Gelenkabschnitt (1200) einschließt;
eine Endeffektoranordnung (140), die sich distal von der Welle erstreckt;
erste, zweite, dritte und vierte Gelenkkabel (138), die an den Gelenkabschnitt wirkgekoppelt sind und sich proximal durch die Welle in das Gehäuse zu proximalen Endabschnitten davon erstrecken; und
die Gelenkanordnung nach Anspruch 1, wobei die proximalen Endabschnitte der ersten, zweiten, dritten und vierten Gelenkkabel an die ersten, zweiten, dritten bzw. vierten Abtriebszahnräder wirkgekoppelt sind.

## Revendications

1. Ensemble d'articulation pour un instrument chirurgical, comprenant :
un boîtier proximal (1210) comportant un corps cylindrique central (1219b) disposé dans celui-ci et des première, deuxième, troisième et quatrième cavités internes (1216 a-d) définies dans celui-ci et agencées autour du corps cylindrique central ;
des premier et second engrenages d'accouplement (1260a, 1260b) disposés coaxialement autour du corps cylindrique central à l'intérieur du boîtier proximal, dans lequel le premier engrenage d'accouplement est disposé plus distalement et le second engrenage d'accouplement est disposé plus proximalement ;
des premier et second arbres d'entrée (1230, 1240) disposés au moins partiellement à l'intérieur des première et deuxième cavités, respectivement, les premier et second arbres d'entrée comportant des récepteurs proximaux (1232, 1242) s'étendant de manière proximale depuis le boîtier proximal et conçus pour recevoir des première et seconde entrées de rotation, les premier et second arbres d'entrée comportant des premier et second engrenages (1236, 1246) respectifs dans lesquels le premier engrenage est disposé plus distalement et en prise par engrènement avec le premier engrenage d'accouplement et le second engrenage est disposé plus proximalement et en prise par engrènement avec le second engrenage d'accouplement de telle sorte que la première entrée de rotation fait tourner le premier arbre d'entrée pour faire tourner ainsi le premier engrenage d'accouplement et de telle sorte que la seconde entrée de rotation fait tourner le second arbre d'entrée pour faire tourner ainsi le second engrenage d'accouplement ; et
des premier, deuxième, troisième et quatrième engrenages de sortie (1250 a-d) disposés à l'intérieur du boîtier proximal, le premier engrenage de sortie (1250a) étant disposé à l'intérieur de la première cavité (1216a) distalement par rapport au premier arbre d'entrée et en prise d'engrènement avec le premier engrenage d'accouplement, le deuxième engrenage de sortie (1250b) étant disposé à l'intérieur de la deuxième cavité (1216b) distalement par rapport au second arbre d'entrée et en prise d'engrènement avec le second engrenage d'accouplement, le troisième engrenage de sortie (1250c) étant disposé à l'intérieur de la troisième cavité (1216c) et en prise d'engrènement avec le premier engrenage d'accouplement, et le quatrième engrenage de sortie (1250d) étant disposé à l'intérieur de la quatrième cavité (1216d) et en prise d'engrènement avec le second engrenage d'accouplement, dans lequel la première entrée rotative fait tourner les premier et troisième engrenages de sortie dans le même sens avec une amplitude égale et dans lequel la seconde entrée rotative fait tourner les deuxième et quatrième engrenages de sortie dans le même sens avec une amplitude égale.

2. Ensemble d'articulation selon la revendication 1, dans lequel les premier et troisième engrenages de sortie sont diagonalement opposés l'un à l'autre et dans lequel les deuxième et quatrième engrenages de sortie sont diagonalement opposés l'un à l'autre.

3. Ensemble d'articulation selon la revendication 1, dans lequel les premier et second engrenages d'accouplement sont des engrenages composés comportant chacun un engrenage majeur (1262) et un engrenage mineur (1264).

4. Ensemble d'articulation selon la revendication 3, dans lequel l'un des engrenages majeurs ou des engrenages mineurs des premier et second engrenages d'accouplement est disposé en prise avec les premier et second engrenages des premier et second arbres d'entrée, respectivement.

5. Ensemble d'articulation selon la revendication 4, dans lequel l'autre des engrenages majeurs ou des engrenages mineurs des premier et second engrenages d'accouplement est disposé en prise avec les premier et troisième engrenages de sortie et les deuxième et quatrième engrenages de sortie, respectivement.

6. Ensemble d'articulation selon la revendication 1, comprenant en outre des première, deuxième, troisième et quatrième vis mères (1270a-d) s'étendant de manière proximale dans les première, deuxième, troisième et quatrième cavités et fixées en rotation par rapport aux premier, deuxième, troisième et quatrième engrenages de sortie à l'intérieur du boîtier proximal de telle sorte que la rotation de l'un des premier, deuxième, troisième ou quatrième engrenages de sortie fait tourner l'une correspondante des première, deuxième, troisième ou quatrième vis mères.

7. Ensemble d'articulation selon la revendication 6, comprenant en outre des premier, deuxième, troisième et quatrième colliers (1284 a-d) en prise par filetage autour des première, deuxième, troisième et quatrième vis mères, respectivement, de telle sorte que la rotation de l'une des première, deuxième, troisième ou quatrième vis mères déplace en translation l'un correspondant des premier, deuxième, troisième ou quatrième colliers le long de celui-ci.

8. Ensemble d'articulation selon la revendication 7, dans lequel des premier, deuxième, troisième et quatrième câbles d'articulation (138) sont reliés aux premier, deuxième, troisième et quatrième colliers, respectivement, de telle sorte que la translation de l'un des premier, deuxième, troisième ou quatrième colliers tend ou détend l'un correspondant des premier, deuxième, troisième ou quatrième câbles d'articulation.

9. Ensemble d'articulation selon la revendication 6, dans lequel une partie d'arbre proximale (1276) de chacune des première, deuxième, troisième et quatrième vis mères s'étend de manière proximale dans l'une correspondante des première, deuxième, troisième ou quatrième cavités, et supporte l'un correspondant des premier, deuxième, troisième ou quatrième engrenages de sortie sur celle-ci à l'intérieur du boîtier proximal.

10. Ensemble d'articulation selon la revendication 9, dans lequel la partie d'arbre proximale de chacune des première, deuxième, troisième et quatrième vis mères définit une configuration non cylindrique et dans lequel chacun des premier, deuxième, troisième et quatrième engrenages de sortie définit une ouverture (1217) de forme au moins partiellement complémentaire à la partie d'arbre proximale de l'une correspondante des première, deuxième, troisième et quatrième vis mères pour ainsi fixer en rotation les premier, deuxième, troisième et quatrième engrenages de sortie autour des première, deuxième, troisième et quatrième vis mères, respectivement.

11. Instrument chirurgical (50), comprenant :
un boîtier (120) ;
un arbre (130) s'étendant distalement à partir du boîtier, l'arbre comportant une section d'articulation (1200) ;
un ensemble effecteur terminal (140) s'étendant distalement à partir de l'arbre ;
des premier, deuxième, troisième et quatrième câbles d'articulation (138) accouplés de manière fonctionnelle à la section d'articulation et s'étendant de manière proximale à travers l'arbre jusque dans le boîtier jusqu'à des parties d'extrémité proximale de celui-ci ; et
l'ensemble d'articulation selon la revendication 1, dans lequel les parties d'extrémité proximale des premier, deuxième, troisième et quatrième câbles d'articulation sont accouplées de manière fonctionnelle aux premier, deuxième, troisième et quatrième engrenages de sortie, respectivement.
